# EUROPÄISCHE PATENTSCHRIFT

(11) **EP 1 815 875 B1**
(45) Veröffentlichungstag und Bekanntmachung des Hinweises auf die Patenterteilung: **12.08.2015**
(21) Anmeldenummer: 06123627.9
(22) Anmeldetag: 07.11.2006
(51) Int. Cl.: A61L 15/26, B32B 5/32, B32B 27/06, B32B 27/40, B29C 47/02, C08G 18/75, C08G 18/22, C08G 18/48, C08G 18/12, C09J 175/08, B29C 47/88

(54) **WUNDABDECKUNG MIT POLYURETHANHAFTKLEBSTOFF UND DESSEN EXTRUSIONSBESCHICHTUNGSVERFAHREN**
WOUND DRESSING WITH POLYURETHAN PRESSURE ADHESIVE AND ITS EXTRUSION COATING PROCESS
PANSEMENT AVEC UN ADHESIF DE PRESSION A BASE DE POLYURETHANE ET SON PROCÉDÉ DE REVÊTEMENT D'EXTRUSION

(30) Priorität: 06.02.2006 EP 06101315; 27.10.2006 WO PCT/EP2006/010382
(43) Veröffentlichungstag der Anmeldung: 08.08.2007
(73) Patentinhaber: nolax AG, 6203 Sempach Station (CH)
(72) Erfinder: Baron, Catherine, 68890 Meyenheim (FR); Rasche, Alfred, 41539 Dormagen (DE); Leuthard, Fabian, 5634 Merenschwand (CH); Schulthess, Adrian, 1734 Tentlingen (CH); Dobmann, Andreas, 6208 Oberkirch (CH); Leumann, Manuel, 5712 Beinwil am See (CH); Clasen, Dirk, 21643 Beckdorf-Nindorf (DE)
(74) Vertreter: Hepp Wenger Ryffel AG

(56) Entgegenhaltungen:
- EP-A- 1 469 052
- EP-B1- 0 897 406
- WO-A-91/01706
- WO-A-95/15135
- US-A- 6 165 625
- US-B1- 6 524 327
- STOKES VK: "Joining Methods for Plastics and Plastic Composites" POLYMER ENGINEERING AND SCIENCE, Bd. 29, Nr. 19, Oktober 1989 (1989-10), Seiten 1310-1324, XP002418754
- DATABASE WWW [Online] 11. Juli 2003 (2003-07-11), BAYER POLYMERS: "Desmodur / Desmophen für Coatings" XP002419597 gefunden im HTTP://WWW.BAYER-LS.DE/LS/LSWEBCMS.NSF/FIL ES/BROCHURE_DE/$FILE/LS5738_D_DESMODUR_DES MOPHEN.PDF accession no. Desmodur VP LS 2371
- DATABASE WWW [Online] 9. Mai 2005 (2005-05-09), BAYER MATERIALSCIENCE: "Desmodur E 305" XP002419598 gefunden im HTTP://WWW.BAYER-LS.COM accession no. Desmodur E 305

## Beschreibung

Die Erfindung betrifft das Gebiet der auf Schaumstoffmaterialien basierenden Wundabdeckungen, insbesondere Herstellungsverfahren für solche Wundabdeckungen.

Wundabdeckungen sollen eine Vielzahl von Funktionen erfüllen: So z.B. die Gewährleistung der Keimdichtigkeit und die Etablierung eines physiologischen Heilmilieus, wobei hierbei heute bei sekundär heilenden Wunden ein feuchtes Heilmilieu bevorzugt ist. Weiter sollte die Wundabdeckung keine Zytotoxizität oder Allergenität aufweisen und atraumatisch entfernbar/auswechselbar sein. Ferner muss überschüssiges Wundexsudat von der Wunde abgeleitet werden.

In der Wundversorgung finden heute vermehrt Schaumstoff-basierte Wundabdeckungen Verwendung, insbesondere für chronische Wunden. Derartige Schaumstoff-Wundabdeckungen haben den Vorteil, dass Wundexsudat zuverlässig von der Wunde entfernt werden kann aufgrund der Saugfähigkeit des Schaumstoffs. Gegenüber altbekannten Wundabdeckungen wie bspw. Gaze, Mullbinden etc. bieten Schaumstoff-basierte Wundabeckungen also den Vorteil einer grösseren Aufnahmekapazität für Wundexsudat, sowie den weiteren Vorteil, dass kein Verkleben mit der Wunde eintritt und somit ein schmerzfreier Verbandwechsel möglich ist.

Auf der wundabgewandten Seite sind diese Schaumstoff-Wundabdeckungen in der Regel mit einer Keimbarriere, typischerweise einer Folie abgedeckt. Diese Keimbarriere stellt einerseits einen Schutz gegen eindringende Bakterien dar und regelt darüber hinaus auch den Gasaustausch mit der Umgebung. Über eine gezielte Einstellung der Wasserdampfdurchlässigkeit dieser Keimbarriere kann sichergestellt werden, dass unterhalb der Wundabdeckung ein genügend feuchtes Klima herrscht, ohne dass es zu einer Mazeration der Haut kommt. Zudem schützt diese Keimbarriere Kleidung und sonstiges Verbandsmaterial vor austretendem Wundexsudat.

Derzeit werden solche Keimbarrieren, z.B. Folien mit der Schaumstoffunterlage verklebt, indem entweder lösungsmittelbasierte Klebstoffe aufgebracht (typischerweise aufgesprüht) oder auch Heiss-Schmelzklebstoffe aufgetragen werden. Der Klebstoffauftrag kann hierbei entweder einseitig (typischerweise auf die Folie) oder beidseitig, also auf die Folie und den Schaum, erfolgen. Alternativ kann bspw. auch ein wärmeaktivierbares Klebevlies zwischen Folie und Schaumstoff eingelegt werden, wodurch der Verbund bewirkt wird. Es muss jedoch unter Anwendung eines Klebstoffs stets eine Zwischenschicht erzeugt werden, um die Verbindung zwischen Schaumstoff und Folie sicherzustellen.

Kritisch ist hierbei stets, dass die Klebeschicht den Transport von Wasserdampf nicht behindern darf; in der Regel sollte die Durchlässigkeit über 1'000 g / m² / Tag liegen. Eine solche Durchlässigkeit kann bspw. über ein offenes Beschichtungsbild mit Klebstoff oder durch einen vollflächig aufgetragenen, jedoch wasserdampfdurchlässigen Klebstofffilm erreicht werden.

Die Gewährleistung der ausreichenden Wasserdampfdurchlässigkeit durch die Klebstoffschicht stellt in der Praxis während der Herstellung ein nicht zu unterschätzendes Problem dar, während die Durchlässigkeit durch die Folie und den Schaumstoff mit einfachen Routineversuchen ermittelt und eingestellt werden kann. Zudem verteuert die notwendige Klebstoffschicht die Herstellung insgesamt. Auch ist die Verbindung von Folie und Schaumstoff durch eine Klebstoffschicht in der Praxis oftmals nicht überzeugend, da es immer wieder zu Ablösungen der Folie von dem Schaumstoff kommt.

Aus US 5,147,338 ist es bekannt, bei einer Wundabdeckung einen Polyurethan-Film auf einen Polyurethan-Schaumstoff aufzusprühen. Hierdurch kann die o.g. Klebstoffschicht entfallen. Das Aufsprühen eines Polyurethanfilms auf den Schaumstoff hat jedoch eine Vielzahl von Nachteilen: Erstens kann ein gleichmässiger Auftrag nur schwer gewährleistet werden. Zweitens penetriert das aufgesprühte Polyurethanmaterial signifikant in den Schaumstoff, was die Aufnahmefähigkeit des Schaumstoffs für Wundexsudat erheblich senken kann. Zudem müssen, um die Versprühbarkeit von Polyurethanmaterialien zu gewährleisten, Lösungsmittel zugesetzt werden; Lösungsmittelreste in der Wundabdeckung können jedoch nicht toleriert werden.

Aus US 3,668,050 ist ein Operationstuch mit einer Operationsöffnung bekannt. Das Operationstuch kann eine auf ein Schaumstoffmaterial extrudierte Folienschicht aufweisen. Das Schaumstoffmaterial ist auf der wundabgewandten Seite angeordnet und soll u.a. das Abrutschen von abgelegtem Operationsbesteck verhindern.

Aus EP 651 984 ist ein Pflaster bekannt mit einer äusseren, nicht porösen Folienschicht und einer dem Körper zugewandten Haftklebstoffschicht, in welche eine poröse Schicht z.B. aus Schaumstoff mit einer Dicke im Bereich von 0,01 bis 0,5 mm eingebettet ist. Der Haftklebstoff dringt hierbei bis zur äusseren Folienschicht durch; durch die Einbettung der porösen Schicht ist die Anbindung des Haftklebstoffs an das Pflaster fester.

Die Broschüre (Desmodur / Dessnophen für Coatings" von Bayer Polymers (2003-07-11) zeigt aliphatische und aromatische Polyisocyanate (Desmodur®) und Polyacrylat-, Polyester- und Polyetherpolyole (Desmophen®) als deren Reaktionspartner für Zweikomponenten-Polyurethan-Reaktiv-Systeme).

Oftmals ist es gewünscht, die Wundabdeckung wundseitig mit einer Haftklebstoffschicht zu versehen, um ein Verrutschen der Wundabdeckung zu verhindern. Um hierfür geeignet zu sein, sollte ein Haftklebstoff sowohl eine hohe Wasserdampfdurchlässigkeit als auch vorzugsweise eine geringe Wasserabsorption aufweisen.

Es ist daher eine Aufgabe der vorliegenden Erfindung, die Nachteile des bekannten zu vermeiden, insbesondere eine Schaumstoff-Wundabdeckung, ein Herstellungsverfahren für eine Schaumstoff-Wundabdeckung und einen für eine solche Schaumstoff-Wundabdeckung geeigneten Haftklebstoff bereitzustellen, wobei das Verfahren in der Durchführung einfach und kostengünstig ist, keine Rückstände (z.B. Lösungsmittelreste) im fertigen Produkt enthält bzw. hinterlässt und im Ergebnis einen hervorragenden und dauerbelastbaren Verbund zwischen Schaumstoff und Folie sicherstellt, ohne dass die Aufnahmefähigkeit des Schaumstoffs für Wundexsudat herabgesetzt wird.

Diese Aufgabe wird durch eine Wundabdeckung, ein Verfahren zur Herstellung einer solchen Wundabdeckung und einen speziell für eine solche Wundabdeckung geeigneten Haftklebstoff gelöst, wie in den Ansprüchen definiert.

Die erfindungsgemässe Wundabdeckung umfasst eine vorzugsweise lösungsmittelfreie Haftklebstoffschicht enthaltend ein Polymer, welches erhältlich ist durch Polymerisierung von
- Isophorondiisocyanat oder einem modifizierten Isophorondiisocyanat; mit
- 2,3,4,5 oder 6 Hydroxylgruppen aufweisenden Polyetherpolyolen mit OH-Zahlen von 20 bis 112 und einem Ethylenoxid (EO)-Gehalt von ≥ 10 Gew.%;
wobei das Verhältnis von NCO-Gruppen des Isophorondiisocyanats oder modifizierten Isophorondiisocyanats zu den gegenüber den NCO-Gruppen reaktionsfähigen funktionellen Gruppen der Diol-/Polyol-Komponente im Bereich von 0,10 bis 0,90 liegt; und
wobei eine insbesondere unterbruchsfreie Schicht des Haftklebstoffs (C)
- bei einem Flächengewicht von etwa 100 g / m² eine Wasserdampfdurchlässigkeit gemäss DIN EN 13726-2:2000 von ≥ 2'000 g / m² / 24 h; und/oder
- eine Wasserabsorption gemäss DIN EN 13726-1:2000 bei einer Schichtdicke von 0,15 mm von < 5 Gew.%
aufweist.

Das Verfahren zur Herstellung einer Wundabdeckung, umfassend
- eine erste Schicht (A) aus einem Schaumstoffmaterial, aufweisend eine erste Hauptfläche und eine zweite Hauptfläche; und
- eine zweite Schicht (B) als Keimbarriere, welche zweite Schicht (B) unmittelbar an die erste Hauptfläche der ersten Schicht (A) angrenzt,
umfassend die folgenden Schritte:
(a) Bereitstellen des Schaumstoffmaterials der ersten Schicht (A);
(b) Flächiges Auftragen, insbesondere Extrudieren mindestens eines thermoplastischen Materials (2) auf die erste Hauptfläche des Schaumstoffmaterials (A) bei einer Temperatur oberhalb der Erweichungstemperatur des thermoplastischen Materials (2), und Verfestigen des Materials (2) zur zweiten Schicht (B);
weiter umfassend das wenigstens teilweise Aufbringen eines Haftklebstoffs enthaltend ein Polymer, welches erhältlich ist durch Polymerisierung von
- Isophorondiisocyanat oder einem modifizierten Isophorondiisocyanat; mit
- 2, 3, 4, 5 oder 6 Hydroxylgruppen aufweisenden Polyetherpolyolen mit OH-Zahlen von 20 bis 112 und einem Ethylenoxid (EO)-Gehalt von ≥ 10 Gew.%;
wobei das Verhältnis von NCO-Gruppen des Isophorondiisocyanats oder des modifizierten Isophorondiisocyanats zu den gegenüber den NCO-Gruppen reaktionsfähigen funktionellen Gruppen der Diol-/Polyol-Komponente im Bereich von 0,10 bis 0,90 liegt; und
wobei der Haftklebstoff die erste Schicht (A) nicht bis zur ersten Hauptfläche hin durchdringt, und wobei eine insbesondere unterbruchsfreie Schicht des Haftklebstoffs
- bei einem Flächengewicht von etwa 100 g / m² eine Wasserdampfdurchlässigkeit gemäss DIN EN 13726-2:2000 von ≥ 2'000 g / m² / 24 h; und/oder
- eine Wasserabsorption gemäss DIN EN 13726-1:2000 bei einer Schichtdicke von 0,15 mm von < 5 Gew.% aufweist.

Alternativ und/oder ergänzend ist es im Rahmen der Erfindung ebenfalls möglich, die erste Schicht aus einem thermoplastischen Schaumstoffmaterial auf eine Temperatur oberhalb der Erweichungstemperatur zu erhitzen. Thermoplastizität des Materials für die zweite Schicht ist dann nicht zwingend erforderlich. Nachteilig bei Verfahren unter Erwärmung des Schaumstoffmaterials ist jedoch, dass das Zusammenführen mit der zweiten Schicht dann unter Druck erfolgt, was bei nur ungenügend eingestellten Randbedingungen des Prozesses die Struktur des erwärmten Schaumstoffmaterials und damit ggf. die Fähigkeit zur Absorption von Wundexsudat und die Wasserdampfdurchlässigkeit beeinträchtigen könnte.

Unter den beiden "Hauptflächen" des Schaumstoffmaterials der ersten Schicht werden hierbei diejenigen Flächen verstanden, welche beim bestimmungsgemässen Gebrauch als Wundabdeckung im wesentlichen parallel zur abgedeckten Hautoberfläche des Verwundeten verlaufen, und zwar auf der wundabgewandten und der wundzugewandten Seite.

Als "im wesentlichen unterbruchsfrei" werden Flächen des erfindungsgemässen mehrschichtigen Materials verstanden, welche keine Öffnungen aufweisen, die der Etablierung des angestrebten feuchten Milieus unterhalb der Wundabdeckung bei bestimmungsgemässem Gebrauch signifikant zuwider laufen würden. Das Kriterium "im wesentlichen unterbruchsfrei" schliesst also nicht das Vorhandensein bspw. von geeignet dimensionierten Perforationslinien aus, entlang welcher ein Abtrennen von Teilstücken durch Abreissen ermöglicht ist.

Überraschenderweise hat sich herausgestellt, dass sich insbesondere durch das Extrudieren der Keimbarriere, vorzugsweise eines Folienmaterials direkt (also ohne Klebstoff-Zwischenschicht) auf das Schaumstoffmaterial Wundabdeckungen herstellen lassen, welche sowohl eine hervorragende Festigkeit des Verbunds als auch nur eine minimale Penetration des Keimbarrieren- bzw. Folienmaterials in den Schaumstoff aufweisen. Die Aufnahmefähigkeit des Schaumstoffs ist daher nicht negativ beeinflusst, wie es hingegen bei aufgesprühtem Folienmaterial beobachtet wird. Das Material zur Herstellung der Keimbarriere muss für die Extrusion (im Gegensatz zur Sprühtechnik) nicht gelöst vorliegen, wodurch keinerlei Rückstände von Lösungsmitteln in dem fertigen Produkt auftreten. Zudem ist die Herstellung einfach zu handhaben, die Schichtdicke des Auftrags kann mit herkömmlichen Extrusionsanlagen (insbesondere mit gängigen Breitschlitzdüsen) zuverlässig eingestellt und konstant gehalten werden. Bei geeignet gewählten Prozessbedingungen können alternativ oder ergänzend auch andere Verfahren als die Extrusion angewendet werden, bspw. Flammkaschierung, Kalandrierung, Ultraschallverschweissung, etc. Geeignete Verfahren sind dem Fachmann an sich geläufig. Im Rahmen der vorliegenden Erfindung ist jedenfalls darauf zu achten, dass die Prozessbedingungen derart gewählt werden, dass die vorstehend beschriebenen Anforderungen an die Aufnahmefähigkeit des Schaummaterials der ersten Schicht für das Wundexsudat, die Wasserdampfdurchlässigkeit beider Schichten und die Eigenschaft der zweiten Schicht als Keimbarriere nicht beeinträchtigt werden.

In weiteren bevorzugten Ausführungsformen der Erfindung wird als Schaumstoffmaterial ein Polyurethan-Schaumstoff bereitgestellt. Vorzugsweise ist das bereitgestellte Schaumstoffmaterial hydrophil. Weiter bevorzugt ist das Schaumstoffmaterial im wesentlichen offenzellig. Vorzugsweise kann eine durchschnittliche Porengrösse im Bereich von 0,02 mm bis 0,2 mm gewählt werden. Geeignete und besonders bevorzugte hydrophile, polyurethanbasierte Schaumstoffmaterialien sind dem Fachmann bekannt und im Markt erhältlich (bspw. Typ Vivo MCF 03 von Corpura B.V., 4879 NE Etten-Leur, The Netherlands; oder Typ 3014 von Polymer Health Technology, Ebbw Vale, NP23 8XE, United Kingdom).

In einer weiteren vorteilhaften Ausgestaltung der Erfindung wird das Schaumstoffmaterial in einer Dicke zwischen 1 mm bis 10 mm, vorzugsweise zwischen 3 mm und 5 mm, bereitgestellt. Derartige Dicken haben sich als hervorragender Kompromiss zwischen erforderlicher Aufnahmefähigkeit für Wundexsudat und Handhabbarkeit der Wundabdeckung erwiesen.

In besonders vorteilhaften Ausgestaltungen der Erfindung wird das mindestens eine Material in Schritt b) derart auf das Schaumstoffmaterial extrudiert, dass sich eine zweite Schicht mit einer Dicke zwischen 10 µm bis 500 µm, vorzugsweise 20 µm bis 200 µm ausbildet; typischerweise 15 µm bis 100 µm, vorzugsweise 20 µm bis 40 µm. Dicken in den angegebenen Bereichen beeinflussen die Handhabbarkeit der Wundabdeckung wenig bis gar nicht, und das o.g. Steuern der Wasserdampfdurchlässigkeit der resultierenden Folie kann in diesem Dickenbereich an alle in der Praxis auftretenden Anforderungen angepasst werden.

Es wurde als besonders vorteilhaft gefunden, dass die Extrusion des mindestens einen Materials in Schritt b) bei einer Temperatur im Bereich zwischen 150 °C und 240 °C, vorzugsweise zwischen 180 °C und 220 °C, insbesondere zwischen 200 °C und 210 °C erfolgt.

In einer weiteren, besonders bevorzugten Ausführungsform können in Schritt b) zwei Schichten erzeugt werden, insbesondere durch Extrusion zweier Materialien, entweder sequentiell oder mittels Co-Extrusion. Auch das Aufbringen einer ersten Folienschicht mittels Extrusion und einer weiteren Schicht bspw. mittels Sprühen ist möglich. Insbesondere ist es durch die Erfindung ermöglicht, bspw. eine dünne, dem Schaumstoff zugewandte Schicht zu erzeugen, welche optimiert ist für die Haftung auf dem Schaumstoff; Schichtdicken von 5 µm bis 10 µm haben sich hierfür bereits als ausreichend erwiesen. Anschliessend kann als aussen liegende, zweite Schicht noch eine Schicht mit z.B. höherer mechanischer Festigkeit (typischerweise in einer Dicke von etwa 10 µm bis 20 µm) aufgetragen, oder gleichzeitig coextrudiert werden. Auf diese Weise können also bspw. Folienschichten erzeugt werden, welche aus mindestens zwei Teil-Schichten aufgebaut sind, wobei die Folienschicht insgesamt sehr dünn sein kann, jedoch trotzdem eine sehr gute Haftung auf dem Schaumstoffmaterial und ein weiches Griffbild aufweist. Zudem ist es durch die Verwendung zweier unterschiedlicher Schichten auch möglich, die Wasserdampfdurchlässigkeit über die Materialauswahl der zusätzlichen Schicht zu steuern, unabhängig von der Gesamtdicke der Folienschicht.

In einer weiteren Ausführungsform der Erfindung kann das mindestens eine Material während und/oder nach dem Aufbringen, insbesondere der Extrusion in Schritt b) aufgeschäumt werden. Die Aufschäumung eines Materials während und/oder nach der Extrusion ist dem Fachmann an sich geläufig und kann mit konventionellen Mitteln (bspw. durch den Zusatz eines Treibmittels wie Azodicarbonamid oder durch nachträgliche Wärmeeinwirkung) erfolgen. Vorteilhafterweise könnte bspw. eine erste, aufgeschäumte Schicht auf den Schaumstoff extrudiert werden, mit einer geringeren Porengrösse als die darunter liegende Schaumstoffschicht. Anschliessend (oder auch gleichzeitig durch Coextrusion) kann dann die abschliessende Schicht, z.B. eine Folienschicht aufgebracht werden. Durch die somit nicht abrupte, sondern stufenweise Abnahme der Porosität und/oder Übergang von Offen- zu Geschlossenzelligkeit kann eine weitere Verbesserung der Haftung zwischen wundzugewandtem Schaumstoff und aussen liegender Folie erzielt werden, wobei zudem noch eine üblicherweise gewünschte, optisch glattere wundabgewandte Seite resultiert.

Selbstverständlich kann auf der ersten Schicht aus einem Schaumstoffmaterial insbesondere auch durch Extrusion eine zweite, aussen liegende Schicht als Keimbarriere aus einem Schaumstoffmaterial erzeugt werden, z.B. auch ohne zusätzliche, aussen liegende (Folien)Schicht(en). Falls als Keimbarriere also ein Schaumstoff zur Anwendung kommt, so muss es sich hierbei um einen geeigneten, im wesentlichen geschlossenzelligen Schaumstoff handeln, um die Wirkung als Keimbarriere und insbesondere auch die Etablierung des angestrebten feuchten Milieus unterhalb der Wundabdeckung zu gewährleisten. Die Erzeugung eines geschlossenzelligen Schaumstoffs durch und/oder nach der Extrusion ist dem Fachmann geläufig, wie vorstehend dargelegt. Ggf. anzupassende Randbedingungen der Extrusion und/oder der Wärmebehandlung können vom Fachmann leicht in Routineversuchen ermittelt werden.

Besonders bevorzugt wird nach der Extrusion des Materials in Schritt b) das Extrudat mittels einer gekühlten Walze an die erste Schicht bzw. den Schaumstoff angepresst. Hierbei findet jedoch keine bzw. nur eine minimale Penetration des Schaumstoffs statt, weil das Extrudat vorgeformt auf den Schaumstoff auftrifft und insbesondere die Viskosität der Schmelze bereits möglichst hoch ist.

Es ist weiter bevorzugt, dass in Schritt b) ein thermoplastisches Polyurethanmaterial, insbesondere ein Polyetherpolyurethan, bereitgestellt wird. Thermoplastische Polyetherpolyurethane mit Zulassung für den medizinischen Bereich sind dem Fachmann bekannt und im Markt erhältlich. Besonders geeignete thermoplastische Materialien weisen gemäss ISO 1133 einen Melt Flow Index (MFI) zwischen 5 und 50 g / 10 min bei 170 °C und einem Stempelgewicht von 21.6 kg auf.

Weiter vorteilhaft kann auf die zweite Hauptfläche der ersten Schicht wundseitig wenigstens teilweise ein wundverträglicher Haftklebstoff aufgebracht werden. Der Haftklebstoff muss hierbei eine derart eingestellte, niedrige Adhäsion aufweisen, dass ein atraumatisches Entfernen (sog. weiches Abzugsverhalten) von der Wunde ermöglicht ist. Der Haftklebstoff ist hierbei vorzugsweise chemisch derart beschaffen, dass er flächig aufgetragen werden kann ohne die Wasserdampfdurchlässigkeit und die Durchleitung des Wundexsudats auf ein untolerierbares Mass zu drücken. Geeignete Schichtdicken können hierbei vom Fachmann mit Routineversuchen leicht ermittelt werden. Typische Schichtdicken sind 5 µm bis 500 µm, bevorzugt sind 10 µm bis 200 µm. Ein zu tiefes Eindringen in den Schaumstoff ist hierbei jedenfalls zu vermeiden, um die Absorption von Wundexsudat nicht zu behindern. Alternativ wird der Haftklebstoff nur partiell, vorzugsweise punktuell aufgetragen; die erforderliche Durchlässigkeit wird dann über das Auftragsbild erreicht. Bevorzugt ist der Haftklebstoff lediglich in einem Randbereich der Wundabdeckung aufgetragen, welcher bei bestimmungsgemässem Gebrauch ausserhalb des Wundbereichs an der Haut anhaftet. Hierbei kann bspw. ein schmaler Streifen oder eine Anfasslasche am Rand der Wundabdeckung vorgesehen sein, welcher nicht mit Haftklebstoff versehen ist und so ein leichtes Entfernen der Wundabdeckung ermöglicht.

Im Gegensatz zu den in EP 897 406 beschriebenen Poly-urethan-Haftklebstoffen haben sich im Rahmen der vorliegenden Erfindung jedoch solche basierend auf Isophorondiisocyanat als besonders vorteilhaft herausgestellt; unerwarteterweise besitzen diese Polyurethan-Haftklebstoffe eine besonders hohe Wasserdampfdurchlässigkeit bei sehr niedriger Wasserabsorption.

Die Erfindung betrifft also weiter einen Haftklebstoff zur Verwendung auf einer Wundabdeckung wie vorstehend beschrieben. Der lösungsmittelfreie Haftklebstoff umfasst ein Polymer, welches erhältlich ist durch Polymerisierung von
- Isophorondiisocyanat oder einem modifizierten Isophorondiisocyanat mit
- 2, 3, 4, 5 oder 6 Hydroxylgruppen aufweisenden Polyetherpolyolen mit OH-Zahlen von 20 bis 112 und einem Ethylenoxid (EO)-Gehalt von ≥ 10 Gew.%;
wobei das Verhältnis von NCO-Gruppen des Isophorondiisocyanats oder modifizierten Isophorondiisocyanats zu den gegenüber den NCO-Gruppen reaktionsfähigen funktionellen Gruppen der Diol-/Polyol-Komponente im Bereich von 0,10 bis 090 liegt; und
wobei eine insbesondere unterbruchsfreie Schicht des Haftklebstoffs
- bei einem Flächengewicht von etwa 100 g / m² eine Wasserdampfdurchlässigkeit gemäss DIN EN 13726:2000 von ≥ 2'000 g / m² / 24 h; und/oder
- eine Wasserabsorption gemäss DIN EN 13726:1:2000 bei einer Schichtdicke von 0,15 mm von < 5 Gew.%
aufweist.

Als Bestandteile des Haftklebstoffs können verwendet werden:

### Diol- oder Polyolverbindungen

Einsetzbar sind: 2, 3, 4, 5 oder 6 Hydroxylgruppen aufweisende Polyetherpolyole mit OH-Zahlen von 20 bis 112 und einem Ethylenoxid-Gehalt von ≥ 10 Gew.%, vorzugsweise 10 bis 40 Gew.%, besonders bevorzugt 10 bis 20 Gew.%. Die Glasübergangstemperaturen sollten hierbei möglichst tief, also unter etwa 20 °C, vorzugsweise unter etwa 0 °C, besonders bevorzugt unter etwa -10 °C.

Polyetherpolyole mit Molekulargewichten zwischen 600 und 12'000 sind bevorzugt und können nach bekannten Verfahren bspw. durch Umsetzung der Starterverbindungen mit einem reaktiven H-Atom mit Alkylenoxiden (beispielsweise Ethylen-, und/oder Propylenoxid, vorzugsweise Propylenoxid, Butylenoxid, Styroloxid, Tetrahydrofuran oder Epichlorhydrin oder Gemischen aus zwei oder mehr davon) erhalten werden. Ebenfalls sind Tetramethylenetherglykole einsetzbar. Ebenfalls möglich sind weitere Modifikationen z.B. mit Monoethylenglycol (MEG), Dipropylenglykol (DPG), Trimethylolpropan (TMP). Bevorzugt für den Einsatz in der Medizin werden heute aliphatische Polyetherpolyole eingesetzt.

Geeignete Startverbindungen sind beispielsweise Wasser, Ethylenglykol, Propylenglykol-1,2 oder -1,3, Butylenglykol-1,4 oder - 1,3, Hexandiol-1,6, Octandiol-1,8, Pentandiol-1,5, Heptandiol-1,7, und deren höhere Homologe, Neopentylglykol, 1,4-Hydroxymethyl-cyclohexan, 2-Methyl-1,3-propandiol, Glyzerin, Trimethylolpropan, 2,2-(bis-4,4'-hydroxyphenyl)-Propan, Trimethylolpropan, Glyzerin oder Pentaerythrit, Hexantriol-1,2,6, Butantriol-1,2,4 Trimethylolethan, Mannitol, Sorbitol, Methylglykoside, Zucker, Phenol, Isononylphenol, Resorcin, Hydrochinon, 1,2,2- oder 1,1,2-Tris-(hydroxyphenyl)-ethan, Ammoniak, Methylamin, Ethylendiamin, Tetra- oder Hexamethylenamin, Triethanolamin, Anilin, Phenylendiamin, 2,4- und 2,6-Diaminotoluol und Polyphenylpolymethylenpolyamine, wie sie sich durch Anilin-Formaldehydkondensation erhalten lassen, oder Gemische aus den vorstehenden Startverbindungen davon.

### Isocyanate

Erfindungsgemäss wird Isophorondiisocyanat verwendet, welches sehr gute Wasserdampfdurchlässigkeiten bei insbesondere geringer Wasserabsorption zulässt.

### Präpolymere

Polyurethan-Präpolymere können eingesetzt werden. In der Regel weisen die im Rahmen der vorliegenden Erfindung einsetzbaren Polyurethan-Präpolymeren ein Molekulargewicht von etwa 500 g / mol bis etwa 15'000 g / mol, vorzugsweise etwa 500 g / mol bis etwa 10'000 g / mol, besonderes bevorzugt etwa 700 g / mol bis etwa 4'500 g / mol auf.

### Zusatzstoffe

Gegebenenfalls kann die erfindungsgemässe PUR-Zusammensetzung noch Zusatzstoffe enthalten wie beispielsweise Weichmacher, Stabilisatoren wie Antioxidantien oder Photostabilisatoren, Tackifier, Farbstoffe, Füllstoffe, Verdicker, Rheologieadditive.

### Weichmacher:

Als Weichmacher werden insbesondere Phthalsäurederivate eingesetzt, bspw. Phthalsäureester, welche 6 bis 12 Kohlenstoffatome aufweisen und mit einem linearen Alkanol verestert wurden, z.B. Dioctylphthalat. Polyethylengykole und deren Derivate, pflanzliche und tierische Öle, wie Glycerinester von Fettsäuren und deren Polymerisationsprodukte und Benzoatverbindungen (Benzoat-Weichmacher, beispielsweise Sucrosebenzoat, Diethylenglykoldibenzoat und/oder Diethylenglykolbenzoat, bei dem etwa 50 bis etwa 95% aller Hydroxylgruppen verestert worden sind, Phosphat-Weichmacher, beispielsweise t-Butylphenyldiphenylphosphat, Polyethylenglykole und deren Derivate, beispielsweise Diphenylether von Poly(ethylenglykol), flüssige Harzderivate, beispielsweise der Methylester von hydriertem Harz sind ebenfalls als Weichmacher geeignet. Besonders bevorzugt sind aliphatische Diester wie Adipin- oder Sebacin-di-nonylester.

### Stabilisatoren:

Zu den im Rahmen der Erfindung eingesetzten Stabilisatoren (Antioxidantien) zählen gehinderte Phenole wie BHT, Irganox® 1010, 1076, 1330, 1520 (Ciba Speciality Chemicals) sowie Tocopherole. Besonders bevorzugt eingesetzt wird Vitamin E (alpha-Tocopherol). Ebenfalls können polyfunktionelle Phenole sowie schwefel- und phoshorhaltige Verbindungen und/oder 1,3,5-Trimethyl-2,4,6-tris(3,5-di-tert-butyl-4-hydroxybenzyl)benzol; Pentaerythrittetrakis-3-(3,5-di-tert-butyl-4-hydroxyphenyl)propionat; n-Octadecyl-3,5-di-tert-butyl-4-hydroxyphenyl)propionat; 4,4-Methylenbis(2,6-di-tert-butyl-phenol); 4,4-Thiobis(6-tert-butyl-o-cresol); 2,6-Di-tert-butylphenol; 6-(4-Hydroxyphenoxy)-2,4-bis(n-octylthio)-1,3,5-triazin; Di-n-Octadecyl-3,5-di-tert-butyl-4-hydroxybenzyl-phosphonate; 2-(n-Octylthio)ethyl-3,5-di-tert-butyl-4-hydroxybenzoat; und Sorbithexa[3-(3,5-di-tertbutyl-4-hydroxy-phenyl)-propionat] zum Einsatz kommen. Als Photostabilisatoren sind zum Beispiel Tinuvin®-Produkte (Ciba Speciality Chemicals), Benzotriazol-Verbindungen, Salicylate, substituierte Tolyl- und Metall-Chelat-Verbindungen geeignet, wobei Benzotriazol-Derivate bevorzugt werden. Kombinationen der oben genannten Verbindungen sind auch möglich. Die üblicherweise eingesetzten Mengen sind zwischen 0,1 und 10 Gew.%.

Für die Einstellung bestimmter Eigenschaften des Haftklebstoffes können fachüblich weitere Zusatzstoffe verwendet werden. Darunter fallen beispielweise Farbstoffe wie Titandioxid, Füllstoffe wie Talkum, Kreide, Ton und dergleichen. Es ist ebenfalls möglich, bestimmte hydrophile Polymere einzubauen, beispielsweise, PVOH (Polyvinylalkohol), Polyvinylpyrrolidon, Hydroxypropylcellulose, Polyvinylmethylether und Celluloseester, speziell deren Acetate mit geringem Substitutionsgrad. Diese können die Benetzbarkeit der Klebstoffe erhöhen. Unter Füllstoffen werden die in der Polyurethanchemie üblicherweise eingesetzten Füllstoffe verstanden. Darunter zählen auch Zinkoxid, Titanoxid und Kieselsäurederivate (z.B. Aerosile® (Degussa)). Als weitere Zusatzstoff seien beispielsweise die Kurzfasern auf organischer oder anorganischer Basis (z.B. Glasfasern, Textilfasern) genannt.

Um die Benetzung des Untergrundes zu erhöhen können dem PUR-Haftklebstoff übliche Netzmittel zugegeben werden: beispielsweise Poloxamere (Copolymer von Polyoxyethylene and Polyoxypropylene), Sorbitanester, Fettsäuren wie Span.RTM (Sigma-Aldrich), Ester von Polyoxyethylenesorbitan und Fettsäuren, wie Polysorbate oder Polysorbate.RTM (Spectrum Chemical), Polyoxyethylierte hydrierte Castor Öle wie etwa Cremophor.RTM. (BASF), Polyoxyethylene Stearate, z.B. Myrj.RTM. (Uniqema) oder jede Kombination dieser Netzmittel. Vorzugsweise ist das Netzmittel ein Polysorbate und Vitamin E.

### Tackifier:

Zusätzlich kann der Haftklebstoff Tackifierharze enthalten. Es können natürliche, modifizierte natürliche und synthetische Harze eingesetzt werden, typischerweise mit einem Molekulargewicht bis zu 1500 g / mol. Die Kompatibilität der Harze mit den weiteren Komponenten ist jeweils in fachüblichen Routineversuchen zu prüfen. Beispielsweise sind Kohlenwasserstoffharze geeignet, insbesondere C₅- bis C₉-Harze, vorzugsweise mit C₅-Harzen modifizierte C₉-Harze, und dergleichen. Die gesamten Kohlenwasserstoffharze können teil- oder vollhydriert sein. Ebenfalls zum Einsatz kommen Naturharze wie Balsamharz oder Tallharz. Die genannten Harze können auch verestert werden mit entsprechenden polyfunktionellen Alkoholen wie Pentaerythritester, Glycerinester, Diethylenglykolester, Triethylenglykolester oder Methylester und so eingesetzt werden. Bekannte Handelsprodukte sind z.B. "Staybelite" ster 10, "Foral" 85-105, "Hercolyn" D, "Alresen" 214 R, "Alresen" 191 R, "Alresen" 500 R 80 und "Cellolyn" 21 s. Polyterpenharze wie auch die Terpenphenolharze können ebenfalls als Tackifierharze einformuliert werden wie auch die synthetischen Harze: Keton-, Kumaron- und Indenharze und auch Kohlenwasserstoff-Harze sind auch möglich z.B. unter Handelsnamen wie "Ketonharz" N, "Lutonal" J 30, "Lutonal" J 60, "Vinnapas" B 17, "Vinnapas" 50 V 1, Kohlenwasserstoffharz 95 KW 10, KW 20 and KW 30. Polyvinylether ist auch ein wirksamer Tackifier. Acrylatharze können ebenfalls alleine oder in Mischungen mit obengenanten Tackifiern eingesetzt werden.

Eine bevorzugte Ausführungsform der Erfindung betrifft eine Wundabdeckung, umfassend eine erste Schicht aus einem Schaumstoffmaterial, und eine zweite Schicht als Keimbarriere, insbesondere aus einem Folienmaterial, welche zweite Schicht unmittelbar an eine Hauptfläche der ersten Schicht angrenzt, wobei bei einer Porengrösse des im wesentlichen offenzelligen Schaumstoffmaterials im Bereich von 0,02 mm bis 0,2 mm das Material der zweiten Schicht nicht mehr als 0,01 mm in das Schaumstoffmaterial eingedrungen ist. Eine derartige minimale Eindringtiefe lässt sich insbesondere durch ein Verfahren wie vorstehend im Detail dargestellt bewirken, wohingegen bei konventionellem Sprühauftrag von Polyurethan-Lösungen eine Eindringtiefe von mindestens etwa 0,05 mm resultiert.

Eine bevorzugte Ausführungsform der Erfindung betrifft die Verwendung eines Extrusionsverfahrens in der Herstellung einer Folienschicht direkt auf einer Schaumstoff-Wundabdeckung.

Die Erfindung wird nachfolgend anhand von Ausführungsbeispielen und Figuren erläutert, ohne dass der Gegenstand der Erfindung auf diese Ausführungsbeispiele zu beschränken wäre. Es zeigen:
- Fig. 1:: Wundabdeckung mit Schaumstoffschicht und aussenliegender Folie;
- Fig. 2:: Wundabdeckung mit Schaumstoffschicht und zwei aussenliegenden Folienschichten;
- Fig. 3:: Herstellungsverfahren, schematisch;
- Fig. 4:: Wundabdeckung auf einem Arm.

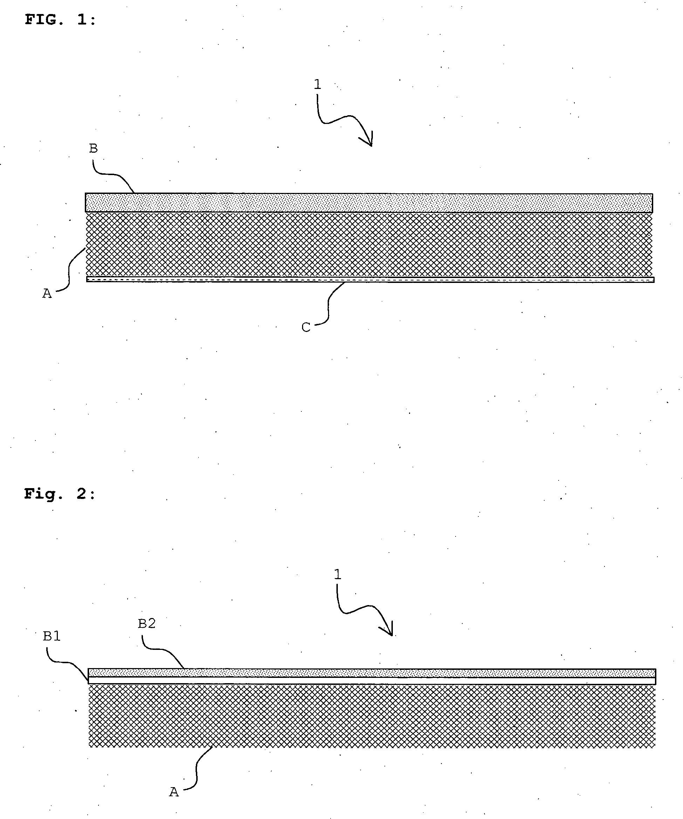

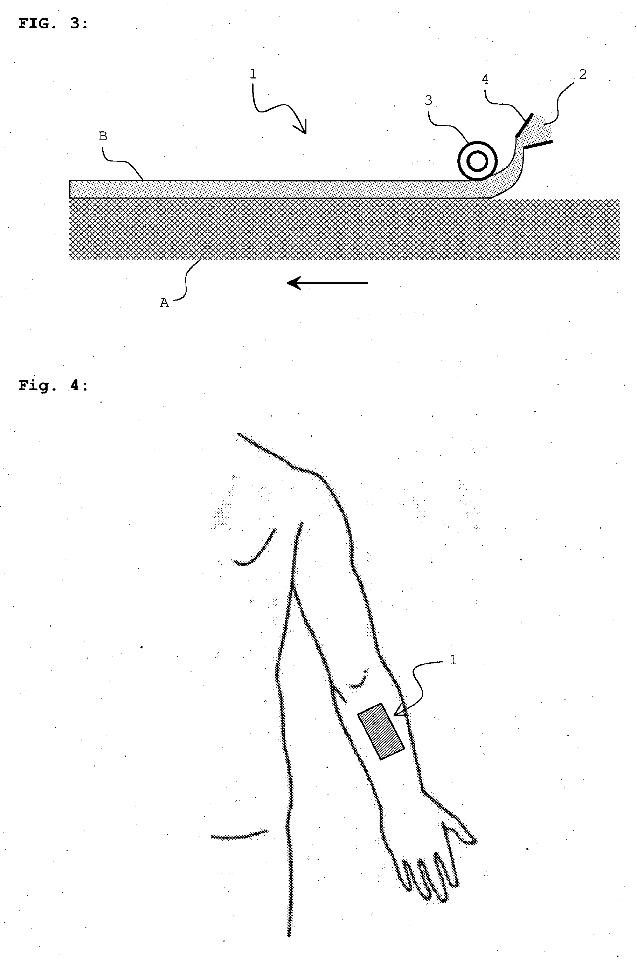

Fig. 1 zeigt schematisch vereinfacht eine erfindungsgemässe Wundabdeckung 1, erhältlich mit dem vorstehend im Detail beschriebenen und in Fig. 3 illustrierten Verfahren, umfassend eine der Wunde zugewandte erste Schicht A aus einem Schaumstoffmaterial, und eine zweite Schicht B als Keimbarriere aus einem Folienmaterial. Die beiden Schichten A und B sind ohne eine Zwischenschicht verbunden; insbesondere liegt keine Klebstoffschicht zwischen der ersten Schicht A und der zweiten Schicht B. Besonders zuverlässige Verbindungen werden erhalten, wenn die Materialien von erster Schicht A und zweiter Schicht B chemisch verwandt sind. Vorteilhaft handelt es sich bei der ersten Schicht A um einen hydrophilen Polyurethanschaumstoff, und bei Schicht B um eine Schicht aus Polyetherpolyurethan; aufgrund der ähnlichen chemischen Natur der beiden Schichten lässt sich eine besonders stabile Verbindung erzielen.

Die Ausführungsform gemäss Fig. 1 weist ferner eine im wesentlichen unterbruchsfrei flächige Schicht C zur Fixierung im Wundbereich auf, welche einen Haftklebstoff enthält. Eine solche Haftklebstoffschicht kann mit fachüblichen Methoden, insbesondere durch Extrusion oder Transferbeschichtung, aufgebracht werden. Selbstverständlich kann das Auftragsbild des Haftklebstoffs auch unterbrochen sein, falls dies für die Gewährleistung einer ausreichenden Wasserdampfdurchlässigkeit erforderlich ist, oder wenn eine Fixierung auf dem Körper bspw. nur in einem Randbereich der Wundabdeckung gewünscht ist.

Fig. 2 zeigt eine Ausführungsform einer erfindungsgemässen Wundabdeckung, bei welcher auf einer ersten Schicht A aus einem Schaumstoffmaterial eine zweite Schicht aufgetragen ist, welche die Teilschichten B1 und B2 umfasst. Die Schichten B1 und B2 können entweder gemeinsam, bspw. mittels Coextrusion (oder sequentieller Extrusion) erzeugt werden. Auch das Erzeugen nur der Schicht B1 mittels Extrusion ist möglich, wobei die Schicht B2 z.B. anschliessend aufgesprüht wird. Die Schicht B2 kann, muss jedoch nicht flächig aufgetragen sein; insbesondere kann es sich hierbei auch um einen Aufdruck handeln, umfassend Schriftzeichen wie Herstellerangaben, Markennamen oder auch Schnittlinien für die Erleichterung eines passgenauen Zuschnitts der Wundabdeckung vor Ort.

Fig. 3 illustriert schematisch eine Ausführungsform des erfindungsgemässen Herstellungsverfahrens anhand einer Wundabdeckung. Eine erste Schicht A eines Schaumstoffmaterials wird in einer herkömmlichen, geeigneten Extrusionsanlage (nicht im Detail gezeigt) entlang der Pfeilrichtung gefördert. Um eine Förderung des Schaumstoffmaterials zuverlässig zu gewährleisten, kann ggf. ein steifes Trägermaterial für die Schicht A vorgesehen werden. Über eine Düse 4, typischerweise eine Breitschlitzdüse, wird ein thermoplastisches Material (hier: ein thermoplastisches Polyetherpolyurethan) extrudiert, typischerweise bei einer Temperatur zwischen 150 °C und 240 °C, vorzugsweise bei 180°C und 220°C. Die Düse 4 hat hierbei keinen direkten Kontakt mit der Schicht A, sondern dass Material 2 verlässt die Düse vorgeformt und wird gewissermassen auf der Schicht A abgelegt und anschliessend vorzugsweise mit einer gekühlten Walze 3 derart an die Schicht A angedrückt, so dass eine feste Verbindung zwischen Schicht A und der aus dem Material 2 gebildeten Schicht B gebildet wird.

Fig. 4 zeigt eine Wundabdeckung 1 gemäss der Erfindung im Gebrauch, hier exemplarisch als Abdeckung einer Wunde an einem Arm. In dieser Draufsicht ist die wundabgewandte Schicht B schraffiert dargestellt. Auf der wundzugewandten Seite befindet sich (in der Figur) verdeckt die Schicht A aus einem Schaumstoffmaterial. Eine Fixierung der Wundabdeckung ist bspw. mit Haftklebestreifen möglich. Bevorzugt ist jedoch, ergänzend oder alternativ, die Befestigung der Wundabdeckung mit einer wundseitigen Haftklebstoffschicht, wie vorstehend beschrieben. Die Grösse der Wundabeckung 1 ist an die jeweilige Wundgrösse angepasst zu wählen, entweder durch Verwendung vorkonfektionierter Wundabdeckungen oder durch Abtrennung (bspw. durch Abreissen entlang von Perforierungen), insbesondere Zuschnitt (bspw. entlang vorzugsweise auf die Schicht A aufgedruckter Markierungen) einer geeignet grosser Wundabdeckung aus einer grösseren Einheit.

Mit dem erfindungsgemässen Verfahren erhaltene Wundverbände wurden auf die Festigkeit des Verbunds zwischen Folie und Schaumstoff analysiert und mit einem derzeit handelsüblichen Produkt verglichen. Als Material für die Herstellung der Folienschicht (Schicht B) wurde Pearlthane^{®} D16N85 (Hersteller Merquinsa), MFI 10 g / 10 min bei 170 °C (Stempelgewicht 21.6 kg), Extrusionstemperatur 205 °C verwendet; die jeweils verwendete Schaumstoffunterlage (Schicht A) ist in Tabelle 1 angegeben. Hierbei wurden die folgenden Testmethoden verwendet:

### Testmethode 1: Haftung

Auf die Polyurethan-Schaumseite von 100 cm² (10 x 10 cm Muster) des Verbunds aus Schicht A und Schicht B werden 5m1 einer wässrigen 0.9 % NaCl-Lösung gegeben. Die befeuchteten Muster werden anschliessend bei 40°C in einer gesättigten Wasserdampfatmosphäre während 24 h gelagert. Nach der Entnahme der Prüflinge werden diese während 10 Minuten auf Raumtemperatur abgekühlt. Anschliessend wird die Haftung zwischen Polyurethan-Folie und Polyurethan-Schaum von Hand qualitativ beurteilt. Zum Vergleich werden Proben beurteilt, welche nicht feucht und warm gelagert wurden. Die gefundene Haftung wird nach einem Notensystem (1 = unbrauchbar, 2 = schwach, 3 = ungenügend, 4 = genügend, 5 = gut, 6 = ausgezeichnet) klassifiziert.

### Testmethode 2: Dichtigkeit

Auf die Polyurethan-Schaumseite von 100 cm² (10 x 10 cm Muster) des Verbunds aus Schicht A und Schicht B werden 5 ml einer wässrigen 0.9 % NaC1-Lösung, welche mit 0.1 % Methylenblau eingefärbt ist, gegeben. Nach 1 Stunde (Raumtemperatur) wird die Dichtigkeit auf der Polyurethan-Folienseite (Schicht B) optisch beurteilt. Die gefundene Dichtigkeit wird nach einem Notensystem (1 = unbrauchbar, 2 = schwach, 3 = ungenügend, 4 = genügend, 5 = gut, 6 = ausgezeichnet) klassifiziert.

Die erhaltenen Resultate sind in Tabelle 1 angegeben:

**Tabelle 1: Beurteilung von Haftung und Dichtigkeit erfindungsgemässer Wundverbände.**

| PUR-Film extrudiert auf: | Vivo MCF 03 (Corpura B.V.) | Foam 3014 (Polymer Health Technology) | Referenzmuster 3M Foam Dressing (Art. 90601) |
|---|---|---|---|
| Haftung zwischen Schaum und Film (Blindwert) | 6 | 6 | 5 |
| Haftung zwischen Schaum und Film nach 24h Feuchtlagerung bei 40°C | 5 | 5 | 3-4 |
| Dichtigkeit nach 1 Stunde | 6 | 6 | 6 |

Es ist ersichtlich, dass die Festigkeit des Verbunds zwischen Schicht A (Schaumstoff) und Schicht B (Folie) bei den erfindungsgemässen Wundverbänden bzw. durch das erfindungsgemässe Herstellungsverfahren für einen solchen Wundverband unter typischer Praxisbeanspruchung verbessert ist.

Als besonders vorteilhafte Haftklebstoffe insbesondere zur Verwendung in der Schicht C (vergl. Figur 1) haben sich Polyurethan-Haftklebstoffe auf der Basis von Isophorondiisocyanat und/oder modifiziertem Isophorondiisocyanat erwiesen. Besonders bevorzugt sind Zusammensetzungen, welche enthalten:
Ein Polymer erhältlich durch Polymerisierung von wenigstens
   a) 2 bis 6 Hydroxylgruppen aufweisenden Polyclen, insbesondere Polyetherpolyolen mit OH-Zahlen von 20 bis 112 und einem Ethylenoxid (EO)-Gehalt von ≥ 10 Gew.%, vorzugsweise 10 bis 40 Gew.%, besonders bevorzugt 10 bis 20 Gew.%; und
   b) Isophorondiisocyanat und/oder modifiziertem Isophorondiisocyanat;
   weiter enthaltend:
   c) in dem/den Polyol(en), insbesondere dem/den Polyetherpolyol(en) a) lösliche, sterisch gehinderte Wismut-Verbindungen, insbesondere Wismut-(III)-Carboxylate auf Basis von Carbonsäuren mit 2 bis 18 C-Atomen, vorzugsweise Bi(III)-neodecanoat; sowie
   d) optional Antioxidantien.

Besonders bevorzugt weist die Haftklebstoff-Zusammensetzung ferner auf:
- Ein Produkt der Funktionalitäten der Komponenten a) und b) von mindestens 5,2; und/oder
- Einen Anteil der Komponente c) von 0,005 bis 0,5 Gew.%, bezogen auf die Komponente a); und/oder
- Einen Anteil an Antioxydantien d) im Bereich von 0,1 bis 1 Gew.%, bezogen auf die Komponente a); und/oder
- Ein Verhältnis von NCO-Gruppen der Komponente b) zu den gegenüber den NCO-Gruppen reaktionsfähigen funktionellen Gruppen der Komponente a) im Bereich von 0,10 bis 0,90.

Besonders bevorzugte Haftklebstoffe weisen bspw. die folgenden Zusammensetzungen auf (Angaben in Gew.%):

| **Harz** | **PSA1: Harz** | **PSA2: Harz** |
|---|---|---|
| Polyetherpolyol (Levagel VPKA 8732; Bayer); OHZ 35 | 99,4 | - |
| Trifunktionelles Polypropylenetherpolyol (Desmophen 5034 BT; Bayer) | - | 99,5 |
| DABCO 33LV (Air Products) | - | 0,5 |
| Bi(III)-Katalysator (hier: -neodecanoat; Coscat 83; Cosan Chemical Corporation) | 0.4 | - |
| Stabilisator (hier: Tocopherol) | 0.2 | - |

| **Härter** | **Mischungsverh. Harz:Härter** | **Mischungsverh. Harz:Härter** |
|---|---|---|
| Präpolymer auf Basis (HDI) (Desmodur E305; Bayer); NCO-Gehalt ca. 13 % | - | 2,48:1 |
| Aliphatisches Präpolymer auf Basis (IPDI) (Desmodur VP LS 2371); NCO-Gehalt ca. 3,8 % | 2,34:1 | - |

Die Eigenschaften dieser Zusammensetzung wurden wie folgt bestimmt:
Wasserdampfdurchlässigkeit (DIN EN 13726-2:2002; bestimmt auf Trägermaterial "Nonwoven (Union)"):
   3'304 g / m² / 24 h bei einem Auftragsgewicht von 100 g / m² (PSA1);
   3' 654 g / m² / 24 h bei einem Auftragsgewicht von 63,5 g / m² (PSA2; unter der Annahme linear mit steigender Schichtdicke abnehmender Wasserdampfdurchlässigkeit ergibt sich bei einem Auftragsgewicht von 100 g / m² im Vergleich zu PSA 1 eine Wasserdampfdurchlässigkeit von 2'320 g / m² / 24 h).
Wasserabsorption (DIN EN 13726-1:2002):
   < 10 g / m² / 24 h (< 2 Gew.%) bei einer Dicke der Haftklebstoffschicht von 0,15 mm (PSA1);
   25 g / m² / 24 h (4,3 Gew.%) bei einer Dicke der Haftklebstoffschicht von 0,15 mm (PSA2).

## Patentansprüche

1. Flächige Wundabdeckung (1), umfassend eine Haftklebstoffschicht enthaltend ein Polymer, welches erhältlich ist durch Polymerisierung von
- Isophorondiisocyanat oder einem modifizierten Isophorondiisocyanat mit
- 2, 3, 4, 5 oder 6 Hydroxylgruppen aufweisenden Polyetherpolyolen mit OH-Zahlen von 20 bis 112 und einem Ethylenoxid (EO)-Gehalt von ≥ 10 Gew.%;
wobei das Verhältnis von NCO-Gruppen des Isophorondiisocynats oder modifizierten Isophorondiisocyanats zu den gegenüber den NCO-Gruppen reaktionsfähigen funktionellen Gruppen der Diol-/Polyol-Komponente im Bereich von 0,10 bis 0,90 liegt; und
wobei eine Schicht des Haftklebstoffs
- bei einem Flächengewicht von 100 g / m² eine Wasserdampfdurchlässigkeit gemäss DIN EN 13726-2: 2000 von ≥ 2'000 g / m² / 24 h und/oder
- eine Wasserabsorption gemäss DIN EN 13726-1: 2000 bei einer Schichtdicke von 0,15 mm von < 5 Gew.%
aufweist.

2. Wundabdeckung gemäss Anspruch 1, umfassend eine erste Schicht (A) aus einem Schaumstoffmaterial und eine zweite Schicht (B) als Keimbarriere, welche zweite Schicht unittelbar an eine Hauptfläche der ersten Schicht (A) angrenzt, **dadurch gekennzeichnet, dass** das Material der zweiten Schicht (B) nicht mehr als 0,01 mm in das Schaumstoffmaterial eingedrungen ist.

3. Verfahren zur Herstellung einer Wundabdeckung (1) gemäss einem der Ansprüche 1 bis 2, aufweisend
- eine erste Schicht (A) aus einem Schaumstoffmaterial, aufweisend eine erste Hauptfläche und eine zweite Hauptfläche; und
- eine zweite Schicht (B) als Keimbarriere, welche zweite Schicht (B) unmittelbar an die erste Hauptfläche der ersten Schicht (A) angrenzt,
umfassend die folgenden Schritte:
(a) Bereitstellen des Schaumstoffmaterials der ersten Schicht (A;
(b) Flächiges Auftragen mindestens eines thermoplastischen Materials (2) auf die erste Hauptfläche des Schaumstoffmaterials (A) bei einer Temperatur oberhalb der Erweichungstemperatur des thermoplastischen Materials (2), und Verfestigen des Materials (2) zur zweiten Schicht (B); weiter umfassend das wenigstens teilweise Aufbringen eines Haftklebstoffs auf die zweite Hauptfläche der ersten Schicht (A), enthaltend ein Polymer, welches erhältich ist durch Polymerisierung von
- Isophorondiisocyanat oder einem modifizierten Isophorondiisocyanat mit
- 2, 3, 4, 5 oder 6 Hydroxylgruppen aufweisenden Polyetherpolyolen mit OH-Zahlen von 20 bis 112 und einem Ethylenoxid (EO)-Gehalt von 2 10 Gew.%;
wobei das Verhältnis von NCO-Gruppen des Isophorondiisocynats oder modifizierten Isophorondiisocyanats zu den gegenüber den NCO-Gruppen reaktionsfähigen funktionellen Gruppen der Diol-/Polyol-Komponente im Bereich von 0,10 bis 0,90 liegt; und
wobei der Haftklebstoff die erste Schicht (A) nicht bis zur ersten Hauptfläche hin durchdringt, und wobei eine Schicht des Haftklebstoffs
- bei einem Flächengewicht von etwa 100 g/m² eine Wasserdampfdurchlässigkeit gemäss DIN EN 13726-2: 2000 von ≥ 2000 g / m² / 24h; und/oder
- eine Wasserabsorption gemäss DIN EN 13726-1: 2000 bei einer Schichtdicke von 0,15 mm von < 5 Gew. %
aufweist; und
wobei die erste Schicht aus einem Schaumstoffmaterial nicht in eine Haftklebstoff-Schicht eingebettet wird.

4. Verfahren gemäss Anspruch 3,
**dadurch gekennzeichnet, dass** als Schaumstoffmaterial der ersten Schicht (A) ein Polyurethan-Schaumstoff bereitgestellt wird.

5. Verfahren gemäss einem der Ansprüche 3 oder 4,
**dadurch gekennzeichnet, dass** die erste Schicht (A) hydrophil ist.

6. Verfahren gemäss einem der Ansprüche 3 bis 5,
**dadurch gekennzeichnet, dass** das Schaumstoffmaterial der ersten Schicht (A) offenzellig ist.

7. Verfahren gemäss einem der Ansprüche 3 bis 6,
**dadurch gekennzeichnet, dass** das mindestens eine Material (2) in Schritt b) derart auf das Schaumstoffmaterial extrudiert wird, dass sich eine zweite Schicht (B) mit einer Dicke zwischen 5 µm bis 500 µm ausbildet.

8. Verfahren gemäss einem der Ansprüche 3 bis 7,
**dadurch gekennzeichnet, dass** die Extrusion des mindestens einen Materials (2) in Schritt b) bei einer Temperatur im Bereich zwischen 150 °C und 240 °C erfolgt.

9. Verfahren gemäss einem der Ansprüche 3 bis 8,
**dadurch gekennzeichnet, dass** in Schritt b) zwei Schichten (B1,B2) der Schicht (B) erzeugt werden, insbesondere durch Extrusion zweier Materialien, entweder sequentiell oder mittels Co-Extrusion.

10. Verfahren gemäss einem der Ansprüche 3 bis 9,
**dadurch gekennzeichnet, dass** das mindestens eine Material (2) während und/oder nach der Extrusion in Schritt b) aufgeschäumt wird.

11. Verfahren gemäss einem der Ansprüche 3 bis 10,
**dadurch gekennzeichnet, dass** nach der Extrusion des Materials (2) in Schritt b) das Extrudat mittels einer gekühlten Walze (3) an die erste Schicht (A) angepresst wird.

12. Verfahren gemäss einem der Ansprüche 3 bis 11,
**dadurch gekennzeichnet, dass** als Material (2) in Schritt b) ein thermoplastisches Polyurethanmaterial, insbesondere ein Polyetherpolyurethan, bereitgestellt wird.

13. Verfahren gemäss Anspruch 1,
wobei die Polyetherpolyole einen Ethylenoxid-Gehalt von 10-40 Gew.%, besonders bevorzugt von 10-20 Gew. %, jeweils mit einem mittleren Molekulargewicht im Bereich von etwa 60C g / mol bis etwa 12'000 g / mol aufweisen.

14. Verfahren gemäss einem der Ansprüche 1 oder 13,
wobei die Polyetherpolyole eine Glasübergangstemperatur T_{g} im Bereich von unter 20 °C aufweisen.

15. Verfahren gemäss einem der Ansprüche 1 oder 13 bis 14, wobei Polyurethan-Präpolymere eingesetzt werden, insbesondere mit einem mittleren Molekulargewicht im Bereich von etwa 500 bis etwa 15'000 g / mol, vorzugsweise von etwa 700 bis etwa 4'500 g.

16. Haftklebstoff zur Verwendung auf einer Wundabdeckung (1), enthaltend ein Polymer, welches erhältlich ist durch Polymerisierung von
- Isophorondiisocyanat oder einem modifizierten Isophorondiisocyanat mit
- 2, 3, 4, 5 oder 6 Hydroxylgruppen aufweisenden Prolyetherpolyolen mit OH-Zahlen von 20 bis 112 und einem Ethylenoxid (EO)-Gehalt von ≥ 10 Gew.%;
wobei das Verhältnis von NCO-Gruppen des Isophorondiisocynats oder modifizierten Isophorondiisocyanats zu den gegenüber den NCO-Gruppen reaktionsfähigen funktionellen Gruppen der Diol-/Polyol-Komponente im Bereich von 0,10 bis 0,90 liegt; und
wobei eine Schicht des Haftklebstoffs
- bei einem Flächengewicht von etwa 100 g / m² eine Wasserdampfdurchlässigkeit gemäss DIN EN 13726-2: 2000 von ≥ 2'000 g / m² / 24 h und/oder
- eine Wasserabsorption gemäss DIN EN 13726-1: 2000 bei einer Schichtdicke von 0,15 mm von < 5 Gew.%
aufweist.

17. Haftklebstoff gemäss Anspruch 16,
wobei die Polyetherpolyole einen Ethylenoxid-Gehalt von 10-40 Gew.%, besonders bevorzugt von 10-20 Gew. %, jeweils mit einem mittleren Molekulargewicht im Bereich von etwa 600 g / mol bis etwa 12'000 g / mol aufweisen.

18. Haftklebstoff gemäss einem der Ansprüche 16 bis 17,
wobei die Polyetherpolyole eine Glasübergangstemperatur T_{g} im Bereich von unter 20 °C aufweisen.

19. Haftklebstoff gemäss einem der Ansprüche 16 bis 18, wobei in den Polyetherpolyolen lösliche, sterisch gehinderte Wismut-Verbindungen, insbesondere Wismut-(III)-Carboxylate auf Basis von Carbonsäuren mit 2 bis 18 C-Atomen, vorzugsweise Bi(III)-neodecanoat; sowie optional Antioxidantien enthalten sind.

20. Haftklebstoff gemäss einem der Ansprüche 16 bis 19, ferner aufweisend:
- ein Produkt der Funktionalitäten des Isophorondiisocyanats bzw. des modifizierten Isophorondiisocyanats und der Polyetherpolyole von mindestens 5,2; und/oder
- einen Anteil eines Katalysators, insbesondere einer sterisch gehinderten Wismut-Verbindung, von 0,005 bis 0,5 Gew.%, bezogen auf die Diol-/Polyol-Komponente; und/oder
- einen Anteil an Antioxydantien im Bereich von 0,1 bis 1 Gew.%, bezogen auf die Diol-/Polyol-Komponente.

21. Haftklebstoff gemäss einem der Ansprüche 16 bis 20, erhältlich unter Verwendung von Polyurethan-Präpolymeren, insbesondere mit einem mittleren Molekulargewicht im Bereich von etwa 500 bis etwa 15'000 g / mol, vorzugsweise von etwa 700 bis etwa 4'500 g.

## Claims

1. Sheetlike wound covering (1), comprising an pressure-sensitive adhesive layer comprising a polymer which is obtainable by polymerizing
- isophorone diisocyanate or a modified isophorone diisocyanate with
- polyether polyols having 2, 3, 4, 5 or 6 hydroxyl groups, OH numbers of 20 to 112 and an ethylene oxide (EO) content of ≥ 10% by weight; wherein the ratio of NCO groups of the isophorone diisocyanate or modified isophorone diisocyanate to the NCO-reactive functional groups of the diol/polyol component is in the range from 0.10 to 0.90; and
wherein a layer of the pressure-sensitive adhesive has
- for a basis weight of 100 g/m² a water vapour transmission rate according to DIN EN 13726-2:2000 of ≥ 2000 g/m²/24 h; and/or
- a water absorption according to DIN EN 13726-1:2000 of < 5% by weight for a layer thickness of 0.15 mm.

2. Wound covering according to Claim 1, comprising a first layer (A) composed of a foam material and a second layer (B) as a germ barrier, this second layer immediately adjoining a major surface of the first layer (A), **characterized in that** the material of the second layer (B) has penetrated not more than 0.01 mm into the foam material.

3. Process for producing a wound covering (1) according to either of Claims 1 and 2, and having
- a first layer (A) composed of a foam material, exhibiting a first major surface and a second major surface; and
- a second layer (B) as a germ barrier, this second layer (B) directly adjoining the first major surface of the first layer (A),
comprising the following steps:
(a) providing the foam material of the first layer (A);
(b) areally applying at least one thermoplastic material (2) to the first major surface of the foam material (A) at a temperature above the softening temperature of the thermoplastic material (2), and solidifying the material (2) to give the second layer (B); further comprising the at least partial application of a pressure-sensitive adhesive to the second major surface of the first layer (A), comprising a polymer which is obtainable by polymerizing
- isophorone diisocyanate or a modified isophorone diisocyanate with
- polyether polyols having 2, 3, 4, 5 or 6 hydroxyl groups, OH numbers of 20 to 112 and an ethylene oxide (EO) content of ≥ 10% by weight; wherein the ratio of NCO groups of the isophorone diisocyanate or modified isophorone diisocyanate to the NCO-reactive functional groups of the diol/polyol component is in the range from 0.10 to 0.90; and wherein the pressure-sensitive adhesive does not penetrate the first layer (A) through to the first major surface, and wherein a layer of the pressure-sensitive adhesive has
- for a basis weight of approximately 100 g/m² a water vapour transmission rate according to DIN EN 13726-2:2000 of ≥ 2000 g/m²/24 h; and/or
- a water absorption according to DIN EN 13726-1:2000 of < 5% by weight for a layer thickness of 0.15 mm; and wherein
the first layer, composed of a foam material, not being embedded into a pressure-sensitive adhesive layer.

4. Process according to Claim 3, **characterized in that** a polyurethane foam is provided as foam material of the first layer (A).

5. Process according to either of Claims 3 and 4, **characterized in that** the first layer (A) is hydrophilic.

6. Process according to any one of Claims 3 to 5, **characterized in that** the foam material of the first layer (A) is open-celled.

7. Process according to any one of Claims 3 to 6, **characterized in that** the at least one material (2) is extruded in step b) onto the foam material in such a way as to form a second layer (B) having a thickness between 5 µm to 500 µm.

8. Process according to any one of Claims 3 to 7, **characterized in that** the extrusion of the at least one material (2) takes place in step b) at a temperature in the range between 150°C and 240°C.

9. Process according to any one of Claims 3 to 8, **characterized in that** two layers (B1, B2) of the layer (B) are produced in step b), more particularly by extrusion of two materials, either sequentially or by means of coextrusion.

10. Process according to any one of Claims 3 to 9, **characterized in that** the at least one material (2) is foamed during and/or after the extrusion in step b).

11. Process according to any one of Claims 3 to 10, **characterized in that**, after the extrusion of the material (2) in step b), the extrudate is pressed against the first layer (A) by means of a cooled roll (3).

12. Process according to any one of Claims 3 to 11, **characterized in that** a thermoplastic polyurethane material, more particularly a polyether polyurethane, is provided as material (2) in step b).

13. Process according to Claim 1, wherein the polyether polyols have an ethylene oxide content of 10-40% by weight, more preferably of 10-20% by weight, in each case having an average molecular weight in the range from about 600 g/mol to about 12 000 g/mol.

14. Process according to either of Claims 1 or 13, wherein the polyether polyols have a glass transition temperature Tg in the region of below 20°C.

15. Process according to any one of Claims 1 or 13 to 14, wherein polyurethane prepolymers are used, more particularly those having an average molecular weight in the range from about 500 to about 15 000 g/mol, preferably from about 700 to about 4500 g/mol.

16. Pressure-sensitive adhesive for use on a wound covering (1), comprising a polymer which is obtainable by polymerizing
- isophorone diisocyanate or a modified isophorone diisocyanate with
- polyether polyols having 2, 3, 4, 5 or 6 hydroxyl groups, OH numbers of 20 to 112 and an ethylene oxide (EO) content of ≥ 10% by weight; wherein the ratio of NCO groups of the isophorone diisocyanate or modified isophorone diisocyanate to the NCO-reactive functional groups of the diol/polyol component is in the range from 0.10 to 0.90; and wherein a layer of the pressure-sensitive adhesive has
- for a basis weight of approximately 100 g/m² a water vapour transmission rate according to DIN EN 13726-2:2000 of ≥ 2000 g/m²/24 h and/or
- a water absorption according to DIN EN 13726-1:2000 of < 5% by weight for a layer thickness of 0.15 mm.

17. Pressure-sensitive adhesive according to Claim 16, wherein the polyether polyols have an ethylene oxide content of 10-40% by weight, more preferably of 10-20% by weight, in each case having an average molecular weight in the range from about 600 g/mol to about 12 000 g/mol.

18. Pressure-sensitive adhesive according to either of Claims 16 and 17, wherein the polyether polyols have a glass transition temperature Tg in the region of below 20°C.

19. Pressure-sensitive adhesive according to any one of Claims 16 to 18, comprising sterically hindered bismuth compounds soluble in the polyether polyols, more particularly bismuth(III) carboxylates based on carboxylic acids having 2 to 18 C atoms, preferably Bi(III) neodecanoate; and also, optionally, antioxidants.

20. Pressure-sensitive adhesive according to any one of Claims 16 to 19, further having:
- a product of the functionalities of the isophorone diisocyanate or of the modified isophorone diisocyanate and of the polyether polyols of at least 5.2; and/or
- a fraction of a catalyst, more particularly of a sterically hindered bismuth compound, of 0.005% to 0.5% by weight, based on the diol/polyol component; and/or
- a fraction of antioxidants in the range from 0.1% to 1% by weight, based on the diol/polyol component.

21. Pressure-sensitive adhesive according to any one of Claims 16 to 20, obtainable using polyurethane prepolymers, particularly those having an average molecular weight in the range from about 500 to about 15 000 g/mol, preferably from about 700 to about 4500 g/mol.

## Revendications

1. Pansement plat (1), comprenant une couche d'adhésif sensible à la pression contenant un polymère qui peut être obtenu par polymérisation
- d'isophorone-diisocyanate ou d'un isophorone-diisocyanate modifié, avec
- des polyétherpolyols comportant 2, 3, 4, 5 ou 6 groupes hydroxy, ayant des indices de groupes OH de 20 à 112 et une teneur en oxyde d'éthylène (OE) de ≥ 10 % en poids ;
le rapport des groupes NCO de l'isophorone-diisocyanate ou de l'isophorone-diisocyanate modifié aux groupes fonctionnels, réactifs vis-à-vis des groupes NCO, du composant diol/polyol se situant dans la plage de 0,10 à 0,90 ; et
une couche de l'adhésif sensible à la pression présentant
- à un poids par unité de surface de 100 g/m² une perméabilité à la vapeur d'eau selon DIN EN 13726-2 : 2000 de ≥ 2 000 g/m²/24 h et/ou
- une absorption d'eau selon DIN EN 13726-1 : 2000 pour une épaisseur de couche de 0,15 mm de < 5 % en poids.

2. Pansement selon la revendication 1, comprenant une première couche (A) d'un matériau alvéolaire et une deuxième couche (B) en tant que barrière aux germes, laquelle deuxième couche est directement adjacente à une surface principale de la première couche (A), **caractérisé en ce que** le matériau de la deuxième couche (B) ne pénètre pas de plus de 0,01 mm dans le matériau alvéolaire.

3. Procédé pour la fabrication d'un pansement (1) selon l'une quelconque des revendications 1 et 2, comportant
- une première couche (A) à base d'un matériau alvéolaire, présentant une première surface principale et une deuxième surface principale ; et
- une deuxième couche (B) en tant que barrière aux germes, laquelle deuxième couche (B) est directement adjacente à la première surface principale de la première couche (A),
comprenant les étapes suivantes :
a) disposition du matériau alvéolaire de la première couche (A) ;
(b) application sur toute la surface d'au moins une matériau thermoplastique (2) sur la première surface principale du matériau alvéolaire (A) à une température supérieure à la température de ramollissement du matériau thermoplastique (2), et solidification du matériau (2) en la deuxième couche (B) ; en outre comprenant l'application au moins partielle sur la deuxième surface principale de la première couche (A) d'un adhésif sensible à la pression contenant un polymère qui peut être obtenu par polymérisation
- d'isophorone-diisocyanate ou d'un isophorone-diisocyanate modifié, avec
- des polyétherpolyols comportant 2, 3, 4, 5 ou 6 groupes hydroxy, ayant des indices de groupes OH de 20 à 112 et une teneur en oxyde d'éthylène (OE) de ≥ 10 % en poids ;
le rapport des groupes NCO de l'isophorone-diisocyanate ou de l'isophorone-diisocyanate modifié aux groupes fonctionnels, réactifs vis-à-vis des groupes NCO, du composant diol/polyol se situant dans la plage de 0,10 à 0,90 ; et
l'adhésif sensible à la pression ne traversant pas la première couche (A) jusqu'à la première couche principale, et une couche de l'adhésif sensible à la pression présentant
- à un poids par unité de surface d'environ 100 g/m² une perméabilité à la vapeur d'eau selon DIN EN 13726-2 : 2000 de ≥ 2 000 g/m²/24 h et/ou
- une absorption d'eau selon DIN EN 13726-1 : 2000 pour une épaisseur de couche de 0, 15 mm de < 5 % en poids ; et
la première couche à base d'un matériau alvéolaire n'étant pas incluse dans une couche d'adhésif sensible à la pression.

4. Procédé selon la revendication 3,
**caractérisé en ce qu'**on dispose en tant que matériau alvéolaire de la première couche (A) une mousse de polyuréthane.

5. Procédé selon l'une quelconque des revendications 3 et 4,
**caractérisé en ce que** la première couche (A) est hydrophile.

6. Procédé selon l'une quelconque des revendications 3 à 5,
**caractérisé en ce que** le matériau alvéolaire de la première couche (A) est à cellules ouvertes.

7. Procédé selon l'une quelconque des revendications 3 à 6,
**caractérisé en ce que** ledit au moins un matériau (2) dans la couche b) est extrudé sur le matériau alvéolaire de sorte qu'il se forme une deuxième couche (B) ayant une épaisseur comprise entre 5 µm et 500 µm.

8. Procédé selon l'une quelconque des revendications 3 à 7, **caractérisé en ce que** l'extrusion dudit au moins un matériau (2) dans l'étape b) s'effectue à une température dans la plage comprise entre 150 °C et 240 °C.

9. Procédé selon l'une quelconque des revendications 3 à 8,
**caractérisé en ce que** dans l'étape b) sont produites deux couches (B1, B2) de la couche (B), en particulier par extrusion de deux matériaux, soit séquentiellement, soit par co-extrusion.

10. Procédé selon l'une quelconque des revendications 3 à 9,
**caractérisé en ce que** ledit au moins un matériau (2) est transformé en mousse pendant et/ou après l'extrusion dans l'étape b).

11. Procédé selon l'une quelconque des revendications 3 à 10,
**caractérisé en ce qu'**après l'extrusion du matériau (2) dans l'étape b) on presse l'extrudat sur la première couche (A) au moyen d'un cylindre refroidi (3).

12. Procédé selon l'une quelconque des revendications 3 à 11,
**caractérisé en ce que** dans l'étape b) on dispose en tant que matériau (2) un matériau polyuréthane thermoplastique, en particulier un polyéther-polyuréthane.

13. Procédé selon la revendication 1,
dans lequel les polyétherpolyols présentent une teneur en oxyde d'éthylène de 10-40 % en poids, de façon particulièrement préférée de 10-20 % en poids, ayant chacun une masse moléculaire moyenne dans la plage d'environ 600 g/mole à environ 12 000 g/mole.

14. Procédé selon l'une quelconque des revendications 1 et 13,
dans lequel les polyétherpolyols présentent une température de transition vitreuse Tg dans la plage inférieure à 20 °C.

15. Procédé selon l'une quelconque des revendications 1 ou 13 et 14,
dans lequel on utilise des prépolymères polyuréthane, en particulier ayant une masse moléculaire moyenne dans la plage d'environ 500 à environ 15 000 g/mole, de préférence d'environ 700 à environ 4 500 g/mole.

16. Adhésif sensible à la pression pour l'utilisation sur un pansement (1), contenant un polymère qui peut être obtenu par polymérisation
- d'isophorone-diisocyanate ou d'un isophorone-diisocyanate modifié, avec
- des polyétherpolyols comportant 2, 3, 4, 5 ou 6 groupes hydroxy, ayant des indices de groupes OH de 20 à 112 et une teneur en oxyde d'éthylène (OE) de ≥ 10 % en poids ;
le rapport des groupes NCO de l'isophorone-diisocyanate ou de l'isophorone-diisocyanate modifié aux groupes fonctionnels, réactifs vis-à-vis des groupes NCO, du composant diol/polyol se situant dans la plage de 0,10 à 0, 90 ; et
une couche de l'adhésif sensible à la pression présentant
- à un poids par unité de surface d'environ 100 g/m² une perméabilité à la vapeur d'eau selon DIN EN 13726-2 : 2000 de ≥ 2 000 g/m²/24 h et/ou
- une absorption d'eau selon DIN EN 13726-1 : 2000 pour une épaisseur de couche de 0,15 mm de < 5 % en poids.

17. Adhésif sensible à la pression selon la revendication 16,
dans lequel les polyétherpolyols présentent une teneur en oxyde d'éthylène de 10-40 % en poids, de façon particulièrement préférée de 10-20 % en poids, ayant chacun une masse moléculaire moyenne dans la plage d'environ 600 g/mole à environ 12 000 g/mole.

18. Adhésif sensible à la pression selon l'une quelconque des revendications 16 et 17,
dans lequel les polyétherpolyols présentent une température de transition vitreuse Tg dans la plage inférieure à 20 °C.

19. Adhésif sensible à la pression selon l'une quelconque des revendications 16 à 18, dans lequel sont contenus des composés de bismuth à encombrement stérique, solubles dans les polyétherpolyols, en particulier des carboxylates de bismuth-(III) à base d'acides carboxyliques ayant de 2 à 18 atomes de carbone, de préférence du néodécanoate de Bi-(III), ainsi que d'optionnels antioxydants.

20. Adhésif sensible à la pression selon l'une quelconque des revendications 16 à 19, comportant en outre :
- un produit des fonctionnalités de l'isophorone-diisocyanate ou de l'isophorone-diisocyanate modifié et des polyétherpolyols d'au moins 5,2 ; et/ou
- une proportion d'un catalyseur, en particulier d'un composé de bismuth à encombrement stérique, de 0,005 à 0,5 % en poids, par rapport au composant diol/polyol et/ou
- une proportion d'antioxydants dans la plage de 0,1 à 1 % en poids, par rapport au composant diol/polyol.

21. Adhésif sensible à la pression selon l'une quelconque des revendications 16 à 20,
pouvant être obtenu avec utilisation de prépolymères polyuréthane, en particulier ayant une masse moléculaire moyenne dans la plage d'environ 500 à environ 15 000 g/mole, de préférence d'environ 700 à environ 4 500 g/mole.
